# EUROPEAN PATENT SPECIFICATION

(11) **EP 0 636 362 B1**
(45) Date of publication and mention of the grant of the patent: **22.12.1999**
(21) Application number: 94115260.5
(22) Date of filing: 22.03.1991
(51) Int. Cl.: A61K 9/00, A61K 9/12

(54) **The use of soluble fluorosurfactants for the preparation of metered-dose aerosol formulations**
Anwendung von löslichen fluorhaltigen Tensiden zur Herstellung von Aerosol-Präparaten mit dosierter Abgabe
Emploi de tensio-actifs fluorés solubles dans la préparation de compositions pour aérosol doseur

(30) Priority: 23.03.1990 US 498333
(43) Date of publication of application: 01.02.1995
(62) Divisional of application: 91907436.9
(73) Proprietor: MINNESOTA MINING AND MANUFACTURING COMPANY, St. Paul, Minnesota 55133-3427 (US)
(72) Inventor: Schultz, Robert K., St Paul, Minnesota 55133-3427 (US); Quessy, Stephen N., St Paul, Minnesota 55133-3427 (US)
(74) Representative: Bowman, Paul Alan

(56) References cited:
- WO-A-90/07333
- WO-A-91/11173
- US-A- 3 250 808
- US-A- 4 352 789
- CHEMICAL ABSTRACTS, vol. 89, no. 14, 2 October 1978, Columbus, Ohio, US; abstract no. 117545k, & JP-A-53 031 582 (DAIKIN KOGYO CO.,LTD.) 24 March 1978

## Description

### TECHNICAL FIELD OF THE INVENTION

This invention relates to suspension aerosol formulations suitable for the administration of medicaments. More particularly, it relates to pharmaceutical suspension aerosol formulations using 1,1,1,2-tetrafluoroethane or 1,1,1,2,3,3,3-heptafluoropropane as the propellant.

### BACKGROUND OF THE INVENTION

Pharmaceutical suspension aerosol formulations currently use a mixture of liquid chlorofluorocarbons as the propellant. Fluorotrichloromethane, dichlorodifluoromethane and dichlorotetrafluoroethane are the most commonly used propellants in aerosol formulations for administration by inhalation.

Chlorofluorocarbons have been implicated in the destruction of the ozone layer and their production is being phased out. Hydrofluorocarbon 134a (HFC-134a, 1,1,1,2-tetrafluoroethane) and hydrofluorocarbon 227 (HFC-227, 1,1,1,2,3,3,3,-heptafluoropropane) are viewed as being more ozone friendly than many chlorofluorocarbon propellants; furthermore, they have low toxicity and vapor pressures suitable for use in aerosols.

U.S. Pat. No. 4,352,789 discloses a self-propelling, powder dispensing aerosol composition comprising between 0.001 and 20 percent by weight of a finely-divided solid material coated with a dry coating of a perfluorinated surface-active dispersing agent of a particular type which constitutes between 0.1 to 20 percent by weight of the coated solid and a halogenated propellant. The solid material can be a medicament. The use of 1,1,1,2-tetrafluoroethane or 1,1,1,2,3,3,3-heptafluoropropane as a propellant is not specifically disclosed. Perfluorinated carboxylic acid surfactants are not disclosed.

### SUMMARY OF THE INVENTION

This invention provides a suspension aerosol formulation, comprising: a propellent comprising a hydrofluorocarbon selected from the group consisting of 1,1,1,2-tetrafluoroethane and 1,1,1,2,3,3,3-heptafluoropropane and a mixture thereof; a therapeutically effective amount of a powdered medicament, and between 0.001 and 0.6 percent by weight based on the total weight of said formulation of a perfluorinated surface-active dispersing agent comprising a perfluorinated carboxylic acid soluble in the propellant; the formulation exhibiting substantially no crystallization of said medicament over a prolonged period, being substantially readily redispersible, and upon redispersion not flocculating so quickly as to prevent reproducible dosing of the medicament.

The pharmaceutical suspension aerosol formulations of the invention are suitable, for example, for dermal, pulmonary, or mucosal (e.g., buccal or nasal) administration.

### DETAILED DESCRIPTION OF THE INVENTION

The term "suspension aerosol" means that the medicament is in powder form and is substantially insoluble in the propellant.

By "prolonged period" as used herein in the context of crystallization is meant at least about four (4) months.

The medicament is micronized, that is, over 90 percent of the particles have a diameter of less than about 10 microns.

The medicament is generally present in an amount effective to bring about the intended therapeutic effect of the medicament. The amount of medicament, however, depends on the potency of the particular medicament being formulated. Generally, the medicament constitutes from 0.01 to 5 percent by weight of the total weight of the formulation, preferably 0.01 to 2 percent by weight of the total weight of the formulation.

Medicaments for delivery by inhalation include, for example, antiallergics, analgesics, bronchodilators, antihistamines, antitussives, anginal preparations, antibiotics, antiinflammatories, hormones, peptides, steroids, enzymes, sulfonamides, or a combination of these.

Examples of medicaments falling within the above therapeutic classes are: isoproterenol hydrochloride or sulfate, phenylephrine bitartrate or hydrochloride, pirbuterol acetate or hydrochloride, disodium cromoglycate, phenylpropanolamine, glucagon, adrenochrome, trypsin, epinephrine bitartrate, ephedrine, narcosine, codeine, atropine, heparin, morphine, albuterol, albuterol sulfate, triamcinolone acetonide, beclomethasone dipropionate, flunisolide, formoterol, salmeterol, colchicine, neomycin, streptomycin, penicillin, tetracycline, chlorotetracycline, hydroxytetracycline, cortisone, hydrocortisone, prednisolone, and insulin.

Preferred medicaments in the practice of this invention include pirbuterol acetate, pirbuterol hydrochloride, disodium cromoglycate, albuterol sulfate, beclomethasone dipropionate, and triamcinolone acetonide.

Perfluorinated surface-active dispersing agents useful in the invention are perfluorinated carboxylic acids or mixture of such acids that are soluble in 1,1,1,2-tetrafluoroethane, 1,1,1,2,3,3,3-heptafluoropropane, or a mixture thereof.

Suitable perfluorinated carboxylic acids are those having the general formula: 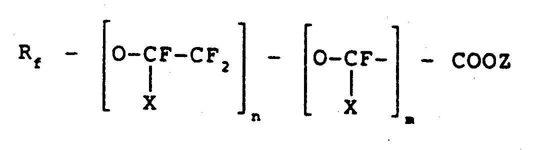 wherein
R_{f} is selected from the group consisting of perfluorinated straight chain, branched chain, or cyclic alkyl or combinations thereof containing three to ten carbon atoms, wherein cyclic alkyl optionally contains one or more catenary oxygen or nitrogen atoms;
each X is independently selected from the group consisting of fluoro and straight chain or branched chain perfluoroalkyl of one to four carbon atoms;
n and m are independently integers from zero to three with the proviso that the sum of n and m is less than or equal to four; and
Z is selected from the group consisting of hydrogen and straight or branched chain alkyl containing one to four carbon atoms.

When m and n are zero, the dispersing agent is perfluoro straight chain, branched chain, cyclic, or a combination thereof, alkanoic acid or ester. Perfluoroalkanoic acids are known and disclosed, e.g., in "Aliphatic Fluorine Compounds", American Chemical Society Monograph Series, Reinhold Publishing Corporation (1958), Chapter VII. Perfluoroalkanoic acid esters are known and disclosed, e.g., in Chapter IX of the same publication.

When either or both of m and n are non-zero, the dispersing agent is an acid- or ester-functional perfluoro mono-, di-, or polyether. Such perfluoroethers are known and disclosed, e.g., in U.S. Pat. Nos. 3,250,808 (Moore et al.).

Particularly preferred perfluorinated carboxylic acids include perfluorobutanoic acid, perfluorooctanoic acid, and perfluorocyclohexylacetic acid.

The perfluorinated surface-active dispersing agent preferably has a solubility of at least 0.1 percent by weight, more preferably at least 0.3 percent by weight and most preferably at least 0.8 percent by weight in the propellant.

The perfluorinated surface-active dispersing agent constitutes from 0.001 to 0.6 percent by weight, preferably 0.005 to 0.5 percent by weight, of the aerosol formulation. The particular preferred amount depends on the particular medicament being formulated and on the particular surface-active dispersing agent being used. It is preferred that the amount of agent used be approximately the minimum needed to provide a suitable suspension.

The hydrofluorocarbon or mixture thereof is preferably the only propellant present in the formulations of the invention. However, one or more other propellants such as propellant 142b (1-chloro-1,1-difluoroethane) can also be present.

The suspension aerosol formulations of the invention can be prepared by first preparing a solution of the perfluorinated surface-active dispersing agent in the propellant and then suspending the medicament in the solution. In order to prepare a formulation, the perfluorinated surface-active dispersing agent is placed in an aerosol vial, a continuous valve is crimped onto the vial and the vial is pressure filled with the propellant. The vial is shaken on an automatic shaker until all of the dispersing agent is in solution. The micronized medicament is then placed in a separate aerosol vial, a continuous valve is crimped onto the vial and the vial is pressure filled with the previously prepared solution. The medicament is then dispersed in the solution by mixing or homogenizing. If the medicament being formulated is moisture sensitive, these steps should be performed in a dehumidified atmosphere using only cry materials and equipment.

The following examples are provided to illustrate the invention.

In the following examples the quality of the aerosol suspension is rated on a scale of 1 to 5 with 1 their respective axis extending parallel to each other andl indicating a "poor" suspension and 5 indicating an "excellent" suspension. A poor suspension is characterized by one or more of the following: it has a rapid rate of settling or separation, it is difficult to redisperse after settling or separation, it forms large flocs quickly, and it exhibits crystal formation. In contrast, an excellent suspension is slow to settle or separate, is easily redispersed, has minimal flocculation, and exhibits no crystallization. Substantially no crystal formation, relative ease of redispersion, and absence of rapid flocculation after redispersion are important properties in order to provide reproducible dosing of the medicament. Absence of substantial crystal formation provides for maximization of the fraction of the dose deliverable to the target area of the lung. Ease of redispersion permits dosing of a uniform suspension. Finally, rapid flocculation results in a large variation in the dose delivered from the aerosol canister. Suspensions exhibiting a rating of 1 or 2 are not considered desirable in terms of an overall balance of properties of degree of crystallization, ease of redispersibility, and nature of any flocculation, whereas ones exhibiting a rating of 3, 4 or 5 are considered desirable and fall within the scope of this invention.

Except as otherwise indicated the propellant in the Examples below is 1,1,1,2-tetrafluoroethane (HFC-134a).

### Example 1

A 78.7 mg portion of perfluorooctanoic acid ("FC-26" from 3M) was placed in a 4 ounce vial, the vial was sealed with a continuous valve then pressure filled with 149.5 g of 1,1,1,2-tetrafluoroethane. The vial was then shaken on an automatic shaker for 15 minutes. The resulting stock solution contained 0.05% by weight of perfluorooctanoic acid. A 100 mg portion of micronized pirbuterol hydrochloride was placed in a 15 cc vial along with 5 ml of glass beads, the vial was sealed with a continuous valve then pressure filled with 20 g of the previously prepared stock solution. The vial was shaken on an automatic shaker for 10 minutes then placed on a WIG-L-BUG™ grinder/mixer for 30 seconds. The resulting suspension contained 0.5% by weight of pirbuterol hydrochloride and had a quality rating of 5 (excellent).

### Examples 2-10

Using the general method of Example 1, a series of suspension aerosol formulations containing 0.5 percent by weight based on the total weight of the formulation of micronized pirbuterol hydrochloride was prepared. Table 1 shows the amount (percent by weight based on the total weight of the formulation) and identity of the surface-active dispersing agent used and the suspension quality rating.

**Table 1**

| Example | Surface-Active Dispersing Agent | Rating |
|---|---|---|
| 2 | 0.002% perfluorooctanoic acid | 1 |
| 3 | 0.006% perfluorooctanoic acid | 4 |
| 4 | 0.01% perfluorooctanoic acid | 4 |
| 5 | 0.3% perfluorooctanoic acid | 5 |
| 6 | 0.006% perfluorobutanoic acid | 5 |
| 7 | 0.012% perfluorobutanoic acid | 5 |
| 8 | 0.059% perfluorobutanoic acid | 5 |
| 9 | 0.310% perfluorobutanoic acid | 5 |
| 10 | 0.507% perfluorobutanoic acid | 5 |

### Examples 11-20

Using the general method of Example 1, a series of suspension aerosol formulations containing 0.5 percent by weight based on the total weight of the formulation of micronized pirbuterol acetate was prepared. Table 2 shows the amount (percent by weight based on the total weight of the formulation) and identity of the surface-active dispersing agent used and the suspension quality rating.

**Table 2**

| Example | Surface-Active Dispersing Agent | Rating |
|---|---|---|
| 11 | 0.002% perfluorooctanoic acid | 1 |
| 12 | 0.006% perfluorooctanoic acid | 2 |
| 13 | 0.01% perfluorooctanoic acid | 2 |
| 14 | 0.05% perfluorooctanoic acid | 3 |
| 15 | 0.3% perfluorooctanoic acid | 3 |
| 16 | 0.006% perfluorobutanoic acid | 2 |
| 17 | 0.012% perfluorobutanoic acid | 2 |
| 18 | 0.059% perfluorobutanoic acid | 2 |
| 19 | 0.310% perfluorobutanoic acid | 2 |
| 20 | 0.507% perfluorobutanoic acid | 2 |

### Examples 21-29

Using the general method of Example 1, a series of suspension aerosol formulations containing 0.5 percent by weight based on the total weight of the formulation of micronized albuterol sulfate was prepared. Table 3 shows the amount (percent by weight based on the total weight of the formulation) and identity of the surface-active dispersing agent used and the suspension quality rating.

**Table 3**

| Example | Surface-Active Dispersing Agent | Rating |
|---|---|---|
| 21 | 0.002% perfluorooctanoic acid | 1 |
| 22 | 0.006% perfluorooctanoic acid | 1 |
| 23 | 0.01% perfluorooctanoic acid | 1 |
| 24 | 0.05% perfluorooctanoic acid | 1 |
| 25 | 0.3% perfluorooctanoic acid | 1 |
| 26 | 0.006% perfluorobutanoic acid | 1 |
| 27 | 0.012% perfluorobutanoic acid | 1 |
| 28 | 0.310% perfluorobutanoic acid | 1 |
| 29 | 0.507% perfluorobutanoic acid | 1 |

### Examples 30-39

Using the general method of Example 1, a series of suspension aerosol formulations containing 1.5 percent by weight based on the total weight of the formulation of micronized disodium cromoglycate was prepared. Table 4 shows the amount (percent by weight based on the total weight of the formulation) and identity of the surface-active dispersing agent used and the suspension quality rating.

**Table 4**

| Example | Surface-Active Dispersing Agent | Rating |
|---|---|---|
| 30 | 0.002% perfluorooctanoic acid | 3 |
| 31 | 0.006% perfluorooctanoic acid | 4 |
| 32 | 0.01% perfluorooctanoic acid | 3 |
| 33 | 0.05% perfluorooctanoic acid | 3 |
| 34 | 0.3% perfluorooctanoic acid | 3 |
| 35 | 0.006% perfluorobutanoic acid | 3 |
| 36 | 0.012% perfluorobutanoic acid | 3 |
| 37 | 0.059% perfluorobutanoic acid | 4 |
| 38 | 0.310% perfluorobutanoic acid | 2 |
| 39 | 0.507% perfluorobutanoic acid | 2 |

A preferred disodium cromoglycate formulation is the same as Example 31 above except the drug concentration is 0.5 percent by weight drug. This formulation had a suspension quality rating of 5.

### Examples 40-49

Using the general method of Example 1, a series of suspension aerosol formulations containing 0.5 percent by weight based on the total weight of the formulation of micronized epinephrine bitartrate was prepared. Table 5 shows the amount (percent by weight based on the total weight of the formulation) and identity of the surface-active dispersing agent used and the suspension quality rating.

**Table 5**

| Example | Surface-Active Dispersing Agent | Rating |
|---|---|---|
| 40 | 0.002% perfluorooctanoic acid | 2 |
| 41 | 0.006% perfluorooctanoic acid | 2 |
| 42 | 0.01% perfluorooctanoic acid | 2 |
| 43 | 0.05% perfluorooctanoic acid | 2 |
| 44 | 0.3% perfluorooctanoic acid | 2 |
| 45 | 0.006% perfluorobutanoic acid | 2 |
| 46 | 0.012% perfluorobutanoic acid | 2 |
| 47 | 0.059% perfluorobutanoic acid | 2 |
| 48 | 0.310% perfluorobutanoic acid | 2 |
| 49 | 0.507% perfluorobutanoic acid | 2 |

### Examples 50-62

Using the general method of Example 1, a series of suspension aerosol formulations containing 0.3 percent by weight based on the total weight of the formulation of micronized triamcinolone acetonide was prepared. Table 6 shows the amount (percent by weight based on the total weight of the formulation) and identity of the surface-active dispersing agent used and the suspension quality rating.

**Table 6**

| Example | Surface-Active Dispersing Agent | Rating |
|---|---|---|
| 50 | 0.05% perfluorooctanoic acid | 4 |
| 51 | 0.05% isopropyl perfluorocyclohexanecarboxylate | 2 |
| 52 | 0.05% perfluoro-2-ethoxyethoxyacetic acid | 3 |
| 53 | 0.05% methyl perfluoro-2-ethoxyethoxyacetate | 3 |
| 54 | 0.05% perfluoro-2-butoxypropionic acid | 2 |
| 55 | 0.005% perfluoto-2-butoxypropionic acid | 2 |
| 56 | 0.05% perfluoro-3-butoxypropionic acid | 3 |
| 57 | 0.05% methyl perfluoro-3-butoxypropionate | 3 |
| 58 | 0.05% isopropyl perfluoro-2-butoxyethoxy acetate | 3 |
| 59 | 0.05% perfluoro-2-hexyloxyethoxyacetic acid | 3 |
| 60 | 0.005% perfluoro-2-hexyloxyethoxyacetic acid | 4 |
| 61 | 0.05% perfluoro-3-octyloxypropionic acid | 3 |
| 62 | 0.005% perfluoro-3-octyloxypropionic acid | 3 |

### Examples 63-72

Using the general method of Example 1, a series of suspension aerosol formulations containing 0.5 percent by weight based on the total weight of the formulation of micronized pirbuterol acetate was prepared. Table 7 shows the amount (percent by weight based on the total weight of the formulation) and identity of the surface-active dispersing agent used and the suspension quality rating.

**Table 7**

| Example | Surface-Active Dispersing Agent | Rating |
|---|---|---|
| 63 | 0.05% isopropyl perfluorocyclohexanecarboxylate | 2 |
| 64 | 0.05% perfluorocyclohexylacetic acid | 4 |
| 65 | 0.05% perfluoro-2-ethoxyethoxyacetic acid | 5 |
| 66 | 0.05% methyl perfluoro-2-ethoxyethoxyacetate | 5 |
| 67 | 0.05% perfluoro-2-butoxypropionic acid | 5 |
| 68 | 0.05% perfluoro-3-butoxypropionic acid | 5 |
| 69 | 0.05% methyl perfluoro-3-butoxypropionate | 4 |
| 70 | 0.05% isopropyl perfluoro-2-butoxyethoxyacetate | 5 |
| 71 | 0.05% perfluoro-2-hexyloxyethoxyacetic acid | 5 |
| 72 | 0.05% perfluoro-3-octyloxypropionic acid | 5 |

### Examples 73-76

Using the general method of Example 1, a series of suspension aerosol formulations containing 1.5 percent by weight based on the total weight of the formulation of micronized disodium cromoglycate was prepared. Table 8 shows the amount (percent by weight based on the total weight of the formulation) and identity of the surface-active dispersing agent used and the suspension quality rating.

**Table 8**

| Example | Surface-Active Dispersing Agent | Rating |
|---|---|---|
| 73 | 0.05% isopropyl perfluorocyclohexanecarboxylate | 2 |
| 74 | 0.05% perfluoro-2-butoxypropionic acid | 5 |
| 75 | 0.005% perfluoro-2-butoxypropionic acid | 4 |
| 76 | 0.05% isopropyl perfluoro-2-butoxyethoxy acetate | 5 |

### Examples 77-78

Using the general method of Example 1, two suspension aerosol formulations containing 0.5 percent by weight based on the total weight of the formulation of micronized albuterol sulfate were prepared. Table 9 shows the amount (percent by weight based on the total weight of the formulation) and identity of the surface-active dispersing agent used and the suspension quality rating.

**Table 9**

| Example | Surface-Active Dispersing Agent | Rating |
|---|---|---|
| 77 | 0.05% perfluoro-2-butoxypropionic acid | 4 |
| 78 | 0.005% perfluoro-2-butoxypropionic acid | 3 |

### Examples 79-83

Using the general method of Example 1, a series of suspension aerosol formulations containing micronized beclomethasone dipropionate was prepared. Table 10 shows the amount (percent by weight based on the total weight of the formulation) and identity of the surface-active dispersing agent used and the suspension quality rating. In the suspensions of Examples 79-81 the medicament was present in an amount by weight of 0.1% and in those of Examples 82 and 83 it was present in an amount by weight of 0.3%.

**Table 10**

| Example | Surface-Active Dispersing Agent | Rating |
|---|---|---|
| 79 | 0.05% perfluorooctanoic acid | 3 |
| 80 | 0.05% methyl perfluoro-2-ethoxyethoxyacetate | 4 |
| 81 | 0.05% methyl perfluoro-3-butoxypropionate | 4 |
| 82 | 0.05% perfluoro-2-butoxypropionic acid | 2 |
| 83 | 0.005% perfluoro-2-butoxypropionic acid | 2 |

### Examples 84-87

A 10.99 g portion of beclomethasone dipropionate and about 81.8 g of acetone were placed in a 4 ounce glass vial and warmed on a steam bath until a solution was obtained. The solution was divided evenly among four 4 ounce vials each containing approximately 100 mL of 1,1,1,2-tetrafluoroethane. The vials were placed in a refrigerator overnight. The resulting precipitate was collected by filtration then dried under vacuum to provide beclomethasone dipropionate-1,1,1,2-tetrafluoroethane clathrate. The clathrate was micronized using a fluid energy micronizer. Using the general method of Example 1, a series of suspension aerosol formulations containing 0.1% by weight based on the total weight of the formulation of the micronized clathrate was prepared. Table 11 shows the amount (percent by weight based on the total weight of the formulation) and identity of the surface-active dispersing agent used and the suspension quality rating.

**Table 11**

| Example | Surface-Active Dispersing Agent | Rating |
|---|---|---|
| 84 | 0.05% methyl perfluoro-3-butoxypropionate | 5 |
| 85 | 0.05% perfluoro-3-butoxypropionic acid | 5 |
| 86 | 0.05% perfluoro-2-ethoxyethoxyacetic acid | 4 |
| 87 | 0.05% methyl perfluoro-2-ethoxyethoxyacetate | 5 |

### Examples 88-91

A series of aerosol suspension formulations in which 1,1,1,2,3,3,3-heptafluoropropane (HFC-227) serves as the propellant was prepared using the general method of Example 1. Table 12 shows the amount (percent by weight based on the total weight of the formulation) and identity of the surface-active dispersing agent used and the suspension quality rating. The formulations of Examples 88 and 89 contained 0.5 percent by weight based on the total weight of the formulation of micronized pirbuterol acetate. Those of Examples 90 and 91 contained 0.3 percent by weight of micronized triamcinolone acetonide.

**Table 12**

| Example | Surface-Active Dispersing Agent | Rating |
|---|---|---|
| 88 | 0.05% perfluorooctanoic acid | 4 |
| 89 | 0.05% perfluoro-2-butoxypropionic acid | 4 |
| 90 | 0.05% perfluorooctanoic acid | 3 |
| 91 | 0.05% perfluoro-2-butoxypropionic acid | 3 |

## Claims (Claims for the following Contracting State(s): BE, CH, DE, FR, GB, IT, LI, SE)

1. A suspension aerosol formulation, comprising: a propellent comprising a hydrofluorocarbon selected from the group consisting of 1,1,1,2-tetrafluoroethane and 1,1,1,2,3,3,3-heptafluoropropane and a mixture thereof; a therapeutically effective amount of a powdered medicament, and between 0.001 and 0.6 percent by weight based on the total weight of said formulation of a perfluorinated surface-active dispersing agent comprising a perfluorinated carboxylic acid soluble in the propellant; the formulation exhibiting substantially no crystallization of said medicament over a prolonged period, being substantially readily redispersible, and upon redispersion not flocculating so quickly as to prevent reproducible dosing of the medicament.

2. A suspension aerosol formulation according to Claim 1 wherein said agent has a solubility of at least 0.8 percent by weight in the propellant.

3. A suspension aerosol formulation according to Claim 1 wherein said medicament is selected from the group consisting of pirbuterol acetate, pirbuterol hydrochloride, disodium cromoglycate, albuterol sulfate, beclomethasone dipropionate, and triamcinolone acetonide.

4. A suspension aerosol formulation according to Claim 1 comprising 1,1,1,2-tetrafluoroethane as essentially the only propellant.

5. A suspension aerosol formulation according to Claim 1 comprising 1,1,1,2,3,3,3,-heptafluoropropane as essentially the only propellant.

## Claims (Claims for the following Contracting State(s): ES)

1. A method of preparing a suspension aerosol formulation comprising mixing a propellent comprising a hydrofluorocarbon selected from the group consisting of 1,1,1,2-tetrafluoroethane and 1,1,1,2,3,3,3 heptafluoropropane and a mixture thereof; and a therapeutically effective amount of a powdered medicament with between 0.001 and 0.6 percent by weight based on the total weight of said formulation of a perfluorinated surface-active dispersing agent comprising a perfluorinated carboxylic acid soluble in the propellant; the resulting formulation exhibiting substantially no crystallization of said medicament over a prolonged period, being substantially readily redispersible, and upon redispersion not flocculating so quickly as to prevent reproducible dosing of the medicament.

2. A method according to Claim 1 for producing a suspension aerosol formulation wherein said agent has a solubility of at least 0.8 percent by weight in the propellant.

3. A method according to Claim 1 for producing a suspension aerosol formulation wherein said medicament is selected from the group consisting of pirbuterol acetate, pirbuterol hydrochloride, disodium cromoglycate, albuterol sulfate, beclomethasone dipropionate, and triamcinolone acetonide.

4. A method according to Claim 1 for preparing a suspension aerosol formulation wherein said propellant is 1,1,1,2,-tetrafluoroethane.

5. A method according to Claim 1 for preparing a suspension aerosol formulation wherein said propellant is 1,1,1,2,3,3,3,-heptafluoropropane.

## Patentansprüche (Patentansprüche für folgende(n) Vertragsstaat(en): BE, CH, DE, FR, GB, IT, LI, SE)

1. Aerosolformulierung in Form einer Suspension, umfassend: ein Treibmittel, das einen Fluorkohlenwasserstoff umfaßt, der aus 1,1,1,2-Tetrafluorethan und 1,1,1,2,3,3,3-Heptafluorpropan sowie einem Gemisch davon ausgewählt ist; eine therapeutisch wirksame Menge eines pulverisierten Arzneimittels; und zwischen 0,001 und 0,6 Gewichtsprozent, bezogen auf das Gesamtgewicht der Formulierung, eines perfluorierten oberflächenaktiven Dispersionsmittels, umfassend eine in dem Treibmittel lösliche perfluorierte Carbonsäure; wobei die Formulierung über einen längeren Zeitraum hinweg im Wesentlichen keine Kristallisation des Arzneimittels zeigt, im Wesentlichen leicht redispergierbar ist und nach erneuter Dispersion nicht so schnell ausflockt, dass eine reproduzierbare Dosierung des Medikaments verhindert wird.

2. Aerosolformulierung in Form einer Suspension nach Anspruch 1, wobei das Dispersionsmittel in dem Treibmittel eine Löslichkeit von mindestens 0,8 Gewichtsprozent besitzt.

3. Aerosolformulierung in Form einer Suspension nach Anspruch 1, wobei das Arzneimittel aus Pirbuterol-Acetat, Pirbuterol-Hydrochlorid, Dinatrium-Cromoglycat, Albuterol-Sulfat, Beclomethason-Dipropionat und Triamcinolon-Acetonid ausgewählt ist.

4. Aerosolformulierung in Form einer Suspension nach Anspruch 1, wobei 1,1,1,2-Tetrafluorethan im Wesentlichen das einzige Treibmittel ist.

5. Aerosolformulierung in Form einer Suspension nach Anspruch 1, wobei 1,1,1,2,3,3,3-Heptafluorpropan im Wesentlichen das einzige Treibmittel ist.

## Patentansprüche (Patentansprüche für folgende(n) Vertragsstaat(en): ES)

1. Verfahren zur Herstellung einer Aerosolformulierung in Form einer Suspension, umfassend Mischen eines Treibmittels, das einen Fluorkohlenwasserstoff umfaßt, der aus 1,1,1,2-Tetrafluorethan und 1,1,1,2,3,3,3-Heptafluorpropan sowie einem Gemisch davon ausgewählt ist; mit einer therapeutisch wirksamen Menge eines pulverisierten Arzneimittels; und mit zwischen 0,001 und 0,6 Gewichtsprozent, bezogen auf das Gesamtgewicht der Formulierung, eines perfluorierten oberflächenaktiven Dispersionsmittels, umfassend eine in dem Treibmittel lösliche perfluorierte Carbonsäure; wobei die Formulierung über einen längeren Zeitraum hinweg im Wesentlichen keine Kristallisation des Arzneimittels zeigt, im Wesentlichen leicht redispergierbar ist und nach erneuter Dispersion nicht so schnell ausflockt, dass eine reproduzierbare Dosierung des Medikaments verhindert wird.

2. Verfahren nach Anspruch 1 zur Herstellung einer Aerosolformulierung in Form einer Suspension, wobei das Dispersionsmittel in dem Treibmittel eine Löslichkeit von mindestens 0,8 Gewichtsprozent besitzt.

3. Verfahren nach Anspruch 1 zur Herstellung einer Aerosolformulierung in Form einer Suspension, wobei das Arzneimittel aus Pirbuterol-Acetat, Pirbuterol-Hydrochlorid, Dinatrium-Cromoglycat, Albuterol-Sulfat, Beclomethason-Dipropionat und Triamcinolon-Acetonid ausgewählt ist.

4. Verfahren nach Anspruch 1 zur Herstellung einer Aerosolformulierung in Form einer Suspension, wobei 1,1,1,2-Tetrafluorethan das Treibmittel ist.

5. Verfahren nach Anspruch 1 zur Herstellung einer Aerosolformulierung in Form einer Suspension, wobei 1,1,1,2,3,3,3-Heptafluorpropan das Treibmittel ist.

## Revendications (Revendications pour l'(les) Etat(s) contractant(s) suivant(s): BE, CH, DE, FR, GB, IT, LI, SE)

1. Formulation d'aérosol en suspension, comprenant : un agent de propulsion comprenant un hydrofluorocarbone choisi dans le groupe comprenant le 1,1,1,2-tétrafluoroéthane et le 1,1,1,2,3,3,3-heptafluoropropane et leurs mélanges, une quantité thérapeutiquement efficace d'un médicament pulvérulent et entre 0,001 et 0,6% en poids par rapport au poids total de ladite formulation d'un agent dispersant tensioactif perfluoré comprenant un acide carboxylique perfluoré soluble dans l'agent de propulsion, la formulation n'engendrant pratiquement pas de cristallisation du médicament susdit sur une période prolongée, étant sensiblement facilement redispersable et lors de la redispersion ne floculant pas trop rapidement au point d'empêcher tout dosage reproductible du médicament.

2. Formulation d'aérosol en suspension suivant la revendication 1, dans laquelle l'agent susdit a une solubilité d'au moins 0,8 % en poids dans l'agent de propulsion.

3. Formulation d'aérosol en suspension suivant la revendication 1, dans laquelle le médicament est choisi dans le groupe comprenant l'acétate de pirbutérol, le chlorhydrate de pirbutérol, le cromoglycate disodique, le sulfate d'albutérol, le dipropionate de béclométhasone et l'acétonide de triamcinolone.

4. Formulation d'aérosol en suspension suivant la revendication 1, comprenant le 1,1,1,2-tétrafluoroéthane comme essentiellement le seul agent de propulsion.

5. Formulation d'aérosol en suspension suivant la revendication 1, comprenant le 1,1,1,2,3,3,3-heptafluoropropane comme essentiellement le seul agent de propulsion.

## Revendications (Revendications pour l'(les) Etat(s) contractant(s) suivant(s): ES)

1. Procédé de préparation d'une formulation d'aérosol en suspension, comprenant le mélange d'un agent de propulsion comprenant un hydrofluorocarbone choisi dans le groupe comprenant le 1,1,1,2-tétrafluoroéthane et le 1,1,1,2,3,3,3-heptafluoropropane et leurs mélanges, et d'une quantité thérapeutiquement efficace d'un médicament pulvérulent avec entre 0,001 et 0,6% en poids par rapport au poids total de ladite formulation d'un agent dispersant tensioactif perfluoré comprenant un acide carboxylique perfluoré soluble dans l'agent de propulsion, la formulation résultante n'engendrant pratiquement pas de cristallisation du médicament susdit sur une période prolongée, étant sensiblement facilement redispersable et lors de la redispersion ne floculant pas trop rapidement au point d'empêcher tout dosage reproductible du médicament.

2. Procédé suivant la revendication 1, pour produire une formulation d'aérosol en suspension, dans lequel l'agent susdit a une solubilité d'au moins 0,8 % en poids dans l'agent de propulsion.

3. Procédé suivant la revendication 1, pour produire une formulation d'aérosol en suspension, dans lequel le médicament est choisi dans le groupe comprenant l'acétate de pirbutérol, le chlorhydrate de pirbutérol, le cromoglycate disodique, le sulfate d'albutérol, le dipropionate de béclométhasone et l'acétonide de triamcinolone.

4. Procédé suivant la revendication 1, pour préparer une formulation d'aérosol en suspension, dans lequel l'agent de propulsion est le 1,1,1,2-tétrafluoroéthane.

5. Procédé suivant la revendication 1, pour préparer une formulation d'aérosol en suspension dans lequel l'agent de propulsion est le 1,1,1,2,3,3,3-heptafluoropropane.
